Europäisches Patentamt

European Patent Office

Office européen des brevets

(19)

(11) Veröffentlichungsnummer: **0 088 864**
**A2**

(12) # EUROPÄISCHE PATENTANMELDUNG

(21) Anmeldenummer: **83100207.6**

(22) Anmeldetag: **12.01.83**

(51) Int. Cl.³: **A 61 M 16/00**

(30) Priorität: **16.03.82 DE 3209413**

(43) Veröffentlichungstag der Anmeldung:
**21.09.83 Patentblatt 83/38**

(84) Benannte Vertragsstaaten:
**DE FR GB IT NL SE**

(71) Anmelder: **Carl A. Hoyer GmbH**
**Parkallee 44**
**D-2800 Bremen 1(DE)**

(72) Erfinder: **Lasthaus, Hubertus**
**Am Richtsberg 88/1102**
**D-3550 Marburg(DE)**

(74) Vertreter: **Goddar, Heinz, Dr. et al,**
**FORRESTER & BOEHMERT Widenmayerstrasse 4/1**
**D-8000 München 22(DE)**

(54) **Vorrichtung zur hochfrequenten maschinellen Beatmung.**

(57) Vorrichtung zur hochfrequenten maschinellen Beatmung mit einer Steuereinheit zur Regelung des Atemgasgemisches, des Beatmungsdruckes, des Beatmungsvolumens, der Beatmungsfrequenz, sowie einem Anschluß für einen Zuführungskatheter, mit mindestens einem Druck-Reservoir (28) und ein zwischen Druck-Reservoir (28) und dem Anshluß (32) eingebrachtes Steuermittel, dessen Durchgangs-Querschnitt über eine Steuer-Einheit (30) zur Regelung des Gasflusses variiert wird; einem in den Trachealtubus (40) einzuführenden, gegenüber dem Innendurchmesser des Trachealtubus (40) deutlich geringeren Durchmesser aufweisenden Katheter (36), welcher die Beatmungsluft zuführt; und einer Einrichtung zum Erwärmen und Befeuchten der Beatmungsgase.

EP 0 088 864 A2

./...

FIG.1

0088864

Firma Carl A. Hoyer GmbH, Parkallee 44, 2800 Bremen 1
--------------------------------------------------------
Vorrichtung zur hochfrequenten maschinellen Beatmung
--------------------------------------------------------

Die Erfindung betrifft eine Vorrichtung zur hochfrequenten maschinellen Beatmung mit einer Steuereinheit zur Regelung des Atemgasgemisches, des Beatmungsdruckes, des Beatmungsvolumens, der Beatmungsfrequenz und der Atemgasklimatisierung sowie einen Zuführungskatheter.

Apparaturen zur maschinellen Beatmung von Kindern und Erwachsenen während der Narkose und bei Störungen der Lungenfunktion, die eine maschinelle Beatmung notwendig machen, sind bekannt. Dabei werden die Zusammensetzung des Atemgasgemisches, der Beatmungsdruck, das Beatmungsvolumen und die Beatmungsfrequenz kontrolliert.

Konventionell wird dabei mit einer Beatmungsfrequenz
gearbeitet, die im physiologischen Bereich, also deutlich unterhalb von 1/sec., liegt.

Es hat sich gezeigt, daß in vielen Fällen eine Beatmung
mit einer weit höheren Frequenz vorteilhaft sein kann.
Dies gilt insbesondere dann, wenn aus medizinischen
Gründen die Lungen oder der gesamte Brustkorb, die bei
konventioneller Beatmung bewegt werden, ruhig gestellt
werden sollen, und wenn die konventionelle Beatmung, bei
der ein unphysiologischer Überdruck innerhalb der Lunge
entsteht, Schädigungen der Lunge zur Folge hat. Entsprechende Versuche werden seit langem an Experimentaltieren, aber auch in der Klinik durchgeführt, in der
Praxis konnte sich die Hochfrequenz-Beatmung bisher jedoch nicht durchsetzen, weil sie mit hohem apparativem
Aufwand verbunden ist.

Dies ist darauf zurückzuführen, daß die bekannten Beatmungsgeräte, bei denen über einen Balg oder aber über
einen Exzenter der Beatmungsdruck auf- und wieder abgebaut wird, für die Zwecke einer hochfrequenten Beatmung
nicht verwendet werden können, weil ihre mechanische
Trägheit zu groß ist. Weiter stellt sich das Problem,
daß bei einer Hochfrequenz-Ventilation, bei der Frequenzen von deutlich über 100 Imp/min erreicht werden können, die üblichen Trachealtuben nicht zu verwenden sind,
weil das Atemgasvolumen in jedem der nur wenige Millisekunden dauernden Beatmungszyklen geringer ist, als das
Volumen der üblichen Trachealtuben, so daß das Frischgas

0088864

- 3 -

die Lungen nicht erreicht. Die Verwendung eines konventionellen Trachealtubus widerspricht auch dem Prinzip der Hochfrequenz-Ventilation, mit dem jederzeit zur Ausatmung hin offenen System. Nachteilig ist weiterhin, daß bei bekannten Beatmungsgeräten Atemgasanfeuchtungs- und Erwärmungseinrichtungen verwendet werden, die auf Grund ihrer großen Volumina für die Hochfrequenzbeatmung ungeeignet sind.

Die verwendeten Gase, die der kliniksinternen Gasversorgungsanlage oder Gasflaschen entnommen werden, weisen jedoch lediglich Temperaturen von 17-20°C und sehr geringe Luftfeuchtigkeit auf, was ohne besondere Maßnahmen bei Verwendung für die Hochfrequenzbeatmung wegen der Ausschaltung des physiologischen Erwärmungs- und Anfeuchtungsraumes (nämlich dem Nasen- und dem Rachenraum) zu einer Austrocknung d.h. zur Schädigung der Schleimhaut des Patienten und zu einer Auskühlung des gesamten Patienten führen kann. Das bedeutet, daß die Hochfrequenzbeatmung ohne geeignete Maßnahmen zur Erwärmung und Anfeuchtung der Atemgase nicht angewendet werden darf.

Der Erfindung liegt daher die Aufgabe zugrunde, eine Vorrichtung zur maschinellen Beatmung zu schaffen, welche bei verhältnismäßig geringem apparativen Aufwand eine exakte hochfrequente Steuerung der Beatmung gewährleistet; die Beatmungsgase erwärmt und anfeuchtet und eine geeignete Zuführung des Beatmungsgases zum Patienten liefert.

Erfindungsgemäß wird dieses erreicht durch mindestens ein Druck-Reservoir und ein zwischen Druck-Reservoir und dem Ausgang eingebrachtes Steuermittel, dessen Durchgangs-Querschnitt von einer Kontroll-Einheit zur Regelung des Gasflusses variiert wird.

Bei einer bevorzugten Ausführungsform besteht das Steuermittel, dessen Durchgangs-Querschnitt zur Regelung des Gasflusses variiert wird, aus einer Mehrzahl von parallel im Gasfluß liegenden und einzel ansteuerbaren elektro-magnetischen Ventilen.

Vorzugsweise sind mindestens zwei Druck-Reservoirs vorgesehen.

Erfindungsgemäß ist weiter ein in den Trachealtubus einzuführender, ein gegenüber dem Innendurchmesser des Trachealtubus deutlich geringeren Außendurchmesser aufweisender Katheter, welcher die Beatmungsluft zuführt, vorgesehen. Weiter kann vorgesehen sein, daß der Katheter an seinem dem Anschluß entfernten distalen Ende mit einer Fixationseinrichtung versehen ist, die aus drei sternförmig in Schlauchlängsrichtung angeordneten Flügeln besteht, deren Kanten die Innenwandung des Trachealtubus tangieren.

Erfindungsgemäß wird weiter vorgeschlagen, in den Strom des Beatmungsgases, zwischen Ausgang und beatmungsluftzuführenden Katheter, eine Vorrichtung zum Erwärmen und Befeuchten der Beatmungsgase einzubringen.

Vorzugsweise ist vorgesehen, daß die Einrichtung zum Erwärmen und Befeuchten der Beatmungsgase in einer
Mikrodruckkammer integriert ist, eine Zweistoffdüse,
sowie eine beheizte Aufprallfläche aufweist. Auf diese
strahlt die mittig angeordnete Zweistoffdüse das erzeugte Aerosol (Beatmungsluft/Wasser-Gemisch) flächendeckend ab. Dadurch verdampfen die kleinen Wassertröpfchen des Aerosols.

Bei einer bevorzugten Ausgestaltung ist ein Temperaturfühler zur Anzeige der Temperatur des aufgewärmten und
angefeuchteten Beatmungsgases vorgesehen, wobei ein
zweiter Temperaturfühler an der beheizten Aufprallfläche
und ein dritter im Ausgangsatengasstrom der Mikrodruckkammer, zur Regelung der Temperatur des aufgewärmten und
angefeuchteten Beatmungsgases vorgesehen ist.

Weitere Merkmale und Vorteile der Erfindung ergeben sich
aus den Ansprüchen, der Beschreibung und der Zeichnung.
Dabei zeigt:

Fig. 1    ein Blockschaltbild der erfindungsgemäßen Vorrichtung zur hochfrequenten
          maschinellen Beatmung;

Fig. 2    den erfindungsgemäßen Beatmungskatheter
          mit der Fixationseinrichtung zur zen-
          tralen Lagestabilisierung des Beat-
          mungskatheters in einem üblichen Endo-
          trachealtubus;

Fig. 3    die erfindungsgemäße Vorrichtung zum Erwärmen und Befeuchten der Beatmungsgase.

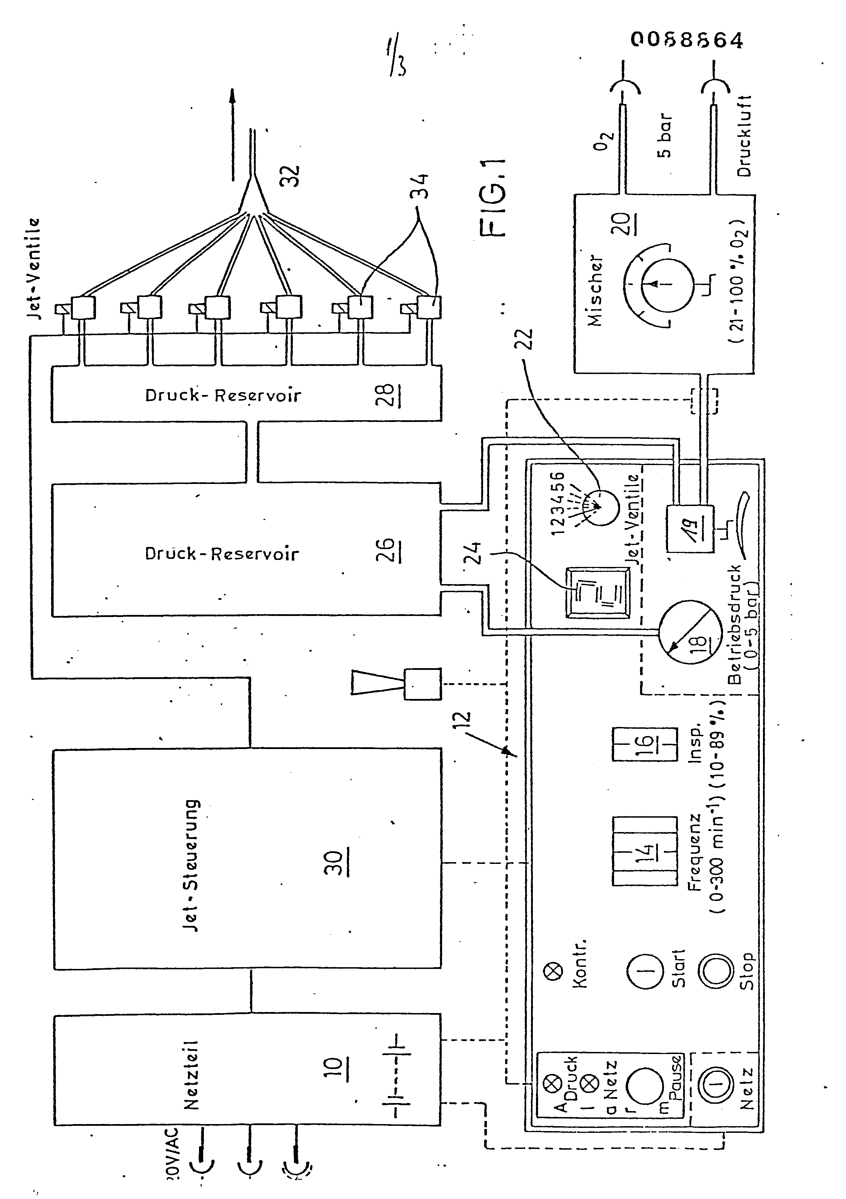

Die Wirkungsweise der erfindungsgemäßen Vorrichtung verdeutlicht Fig. 1. Ein Netzteil 10 versorgt die Kontroll-
und Bedieneinheit 12 mit der erforderlichen Betriebsspannung. Ein Akkumulator dient zur Sicherstellung der
Alarmfunktion bei Netzausfall.

Die Kontroll- und Bedieneinheit 12 weist einen Netzschalter sowie eine Alarmanzeige-Einrichtung auf. Weiter
ist ein Einstell-Organ 14 zur Wahl der Arbeitfrequenz
sowie ein Einstell-Organ 16 zur Wahl des Tastverhältnisses vorgesehen. Weiter ist ein Betriebsdruck-Regler
19 und eine Betriebsdruckanzeige 18 vorgesehen, über die
der gewünschte Arbeitsdruck der über den Mischer 20 von
außen zugeführten Atemgase eingestellt bzw. angezeigt
wird. Die Steuer-Einheit 30 beinhaltet weiter eine Wahleinheit 22 sowie eine Anzeige 24, über die die Anzahl
der angesteuerten Ventile 34 gewählt bzw. angezeigt werden kann. Das erste Druck-Reservoir 26 ist mit einem
zweiten Druck-Reservoir 28 verbunden, von dem aus die
Ventile 34 versorgt werden. Diese Ventile 34 werden von
der Steuer-Einheit 30 angesteuert, welche ihrerseits von
der Kontroll- und Bedieneinheit 12 eingestellt und überwacht werden.

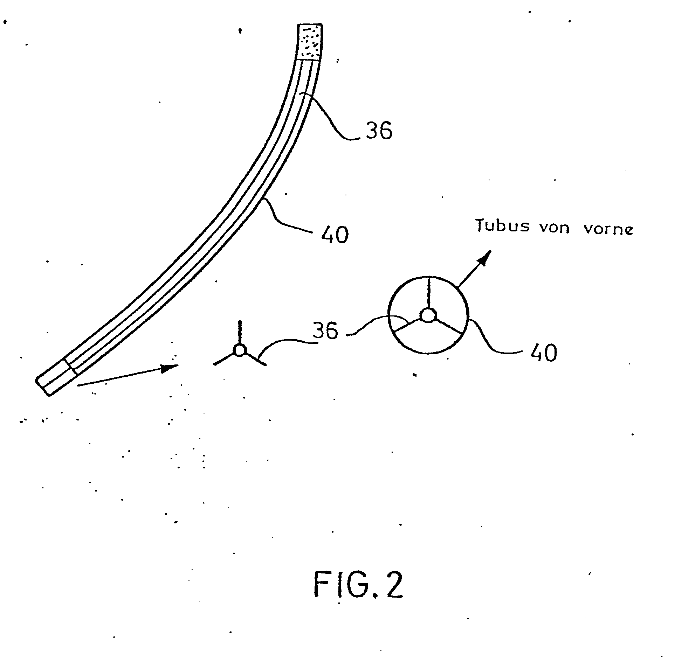

An den Tubus-Anschluß 45 wird der in Fig. 2 gezeigte
Katheter 36 angeschlossen. Dieser Katheter 36 kann in
einen konventionellen Trachealtubus 32 derart eingeführt
werden, daß die sternförmige Fixationseinrichtung eine

zentrale Lage in dem Tubus sicherstellt. Auf diese Weise
ist gewährleistet, daß der Katheter 36 bei Anwendung des
hochfrequent variierenden Beatmungsdruckes keine Lageveränderung besonders bzgl. seiner Abstrahleinrichtung
erfährt.

Das erfindungsgemäße Steuermittel zur Regelung des Gasflusses, dessen Durchgangsquerschnitt von der Kontroll-
und Bedieneinheit 12 über die Steuer-Einheit 30 angesteuert wird, ermöglicht im Zusammenwirken mit dem
Druck-Reservoir 28 bzw. den Druck-Reservoirs 26 und 28,
daß bei konstantem, durch den Regler 19 einstellbaren
Arbeitsdruck, bei über das Einstell-Organ 14 gewählter
Frequenz und bei über das Einstell-Organ 16 gewähltem
Tastverhältnis, das Zeitvolumen des Beatmungsgases in
verschiedenen Stufen definiert werden kann. Falls lediglich ein geringes Zeitvolumen erforderlich ist, wie etwa
bei Kleinkindern, wird lediglich eines der Ventile 34
durch Anwahl der Ziffer 1 an der Wahleinheit 22 aktiviert. Ist dagegen ein großes Gaszeitvolumen erforderlich, so wird der Querschnitt der Steuereinheit größer
gewählt, im Ausführungsbeispiel würden dann also bis zu
alle sechs Ventile 34 aktiviert werden. Die Anzeige 24
verdeutlicht jederzeit die Anzahl der im Betrieb befindlichen Ventile 34.

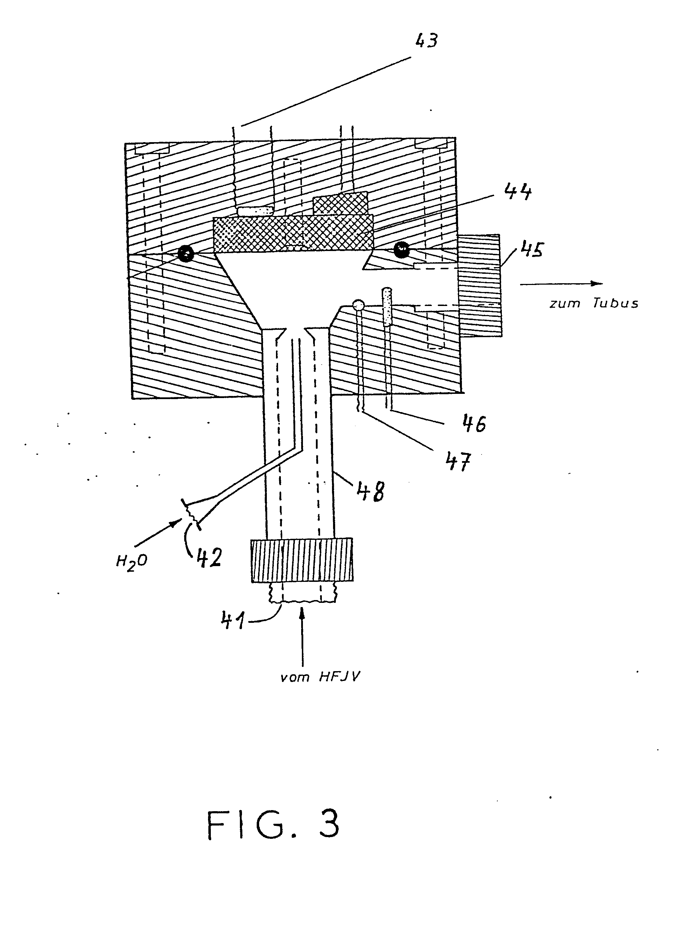

Figur 3 verdeutlicht die erfindungsgemäße Einrichtung
zum Erwärmen und Befeuchten der Beatmungsgase. Das hochfrequent gepulste Beatmungsgas wird über die Gaszuführung 41 der Zweistoffdüse 48 zugeführt, wobei das Wasser
über den Anschluß 42 zugeführt wird. Das durch die Zweistoffdüse erzeugte Aerosol (Wasser/Beatmungsgasgemisch)
wird flächendeckend auf die Aufprallfläche, welche aus
dem Heizelement 44 besteht, abgestrahlt. Dabei verdampfen
die kleinen Wassertröpfchen des Aerosols an der Aufprallfläche, wodurch die Feuchtigkeit und die Temperatur
des Beatmunsgases gleichzeitig erhöht werden.

Zur Regelung der erforderlichen Temperatur ist ein Temperaturfühler 43 vorgesehen, welcher die Temperatur des
elektrischen Heizelementes 44 mißt, sowie ein Temperaturfühler 47 der die Temperatur des befeuchteten Beatmungsgases beim Austritt aus der Mikrodruckkammer erfaßt. Zur
Anzeige der Temperatur, mit der die Beatmungsgase dem
Katheter-Anschluß 45 zugeführt werden, ist ein Temperaturfühler 46 vorgesehen.

Die in der vorstehenden Beschreibung, den Ansprüchen und
der Zeichnung offenbarten Merkmale der Erfindung können
sowohl einzeln als auch in beliebiger Kombination für die
Ausführung der Erfindung in ihren verschiedenen Ausgestaltungen wesentlich sein.

- 9 -

0088864

Bezugszeichenliste
==================

| | | |
|---|---|---|
| 10 | Netzteil | 10 |
| 12 | Kontroll- und Bedieneinheit | 12 |
| 14 | Einstell-Organ | 14 |
| 16 | Einstell-Organ | 16 |
| 18 | Betriebsdruckanzeige | 18 |
| 19 | Betriebsdruckregler | 19 |
| 20 | Mischer | 20 |
| 22 | Wahl-Einheit | 22 |
| 24 | Anzeige | 24 |
| 26 | Druck-Reservoir | 26 |
| 28 | Druck-Reservoir | 28 |
| 30 | Steuer-Einheit | 30 |
| 32 | Ausgang | 32 |
| 34 | Ventile | 34 |
| 36 | Katheter | 36 |
| 38 | Dreizack | 38 |

| 40 | Trachealtubus | 40 |
| 41 | Gaszuführung | 41 |
| 42 | Wasserzuführung | 42 |
| 43 | Temperaturfühler | 43 |
| 44 | Heizelement | 44 |
| 45 | Katheteranschluß | 45 |
| 46 | Temperaturfühler | 46 |
| 47 | Temperaturfühler | 47 |
| 48 | Zweistoffdüse | 48 |

Ansprüche
==================

1. Vorrichtung zur hochfrequenten maschinellen Beatmung mit einer Steuereinheit zur Regelung des Atemgasgemisches, des Beatmungsdruckes, des Beatmungsvolumens, der Beatmungsfrequenz, sowie einem Anschluß für einen Zuführungskatheter, gekennzeichnet durch mindestens ein Druck-Reservoir (28) und ein zwischen Druck-Reservoir (28) und dem Anschluß (32) eingebrachtes Steuermittel, dessen Durchgangs-Querschnitt über eine Steuer-Einheit (30) zur Regelung des Gasflusses variiert wird.

2. Vorrichtung zur hochfrequenten maschinellen Beatmung nach Anspruch 1, dadurch gekennzeichnet, daß das Steuermittel zur Regelung des Gasflusses aus einer Mehrzahl von parallel im Gasfluß liegenden und einzeln ansteuerbaren elektro-magnetischen Ventilen (34) besteht.

3. Vorrichtung zur hochfrequenten maschinellen Beatmung nach Anspruch 1 oder 2, gekennzeichnet durch mindestens
zwei hintereinander liegende Druck-Reservoirs (26, 28).

4. Vorrichtung zur hochfrequenten maschinellen Beatmung
mit einer Steuereinheit zur Kontrolle des Atemgasgemisches, des Beatmungsdruckes, des Beatmungsvolumens,
der Beatmungsfrequenz; einem Anschluß für einen Zuführungskatheter; gekennzeichnet durch einen in den
Trachealtubus (40) einzuführenden, gegenüber dem Innendurchmesser des Trachealtubus (40) deutlich geringeren
Außendurchmesser aufweisenden Katheter (36), welcher
die Beatmungsluft zuführt.

5. Vorrichtung zur hochfrequenten maschinellen Beatmung
nach Anspruch 4, dadurch gekennzeichnet, daß der Katheter (36) an seinem dem Anschluß (32) entfernten distalen
Ende mit einer Fixationseinrichtung versehen ist, die
aus drei sternfömig in Schlauchlängsrichtung angeordneten Flügeln besteht, wobei der Umkreisdurchmesser um
die Flügelkanten dem Innendurchmesser des Endotrachealtubus entspricht.

6. Vorrichtung zur hochfrequenten maschinellen Beatmung
mit einer Steuereinheit zur Kontrolle des Atemgasgemisches, des Beatmungsdruckes, des Beatmungsvolumens,
der Beatmungsfrequenz; einem Anschluß für einen Zuführrungskatheter, gekennzeichnet durch eine Einrichtung
zum Erwärmen und Befeuchten der Beatmungsgase.

7. Vorrichtung nach Anspruch 6, dadurch gekennzeichnet,
daß die Einrichtung zum Erwärmen und Befeuchten der Beatmungsgase eine Zweistoffdüse (48) mit einer Gaszuführung (41) und einem Wasseranschluß (42), sowie eine
beheizte Aufprallfläche aufweist, auf der die kleinen,
in dem zugeführten Wasser/Beatmungsgasgemisch enthaltenen Wasserpartikel verdampfen.

8. Vorrichtung nach Anspruch 7, dadurch gekennzeichnet,
daß die Zweistoffdüse (48) so angeordnet ist, daß das
erzeugte Wasser/Atemgasaerosol flächendeckend auf die
beheizte Aufprallfläche abgestrahlt wird.

9. Vorrichtung nach den Ansprüchen 7 und 8, dadurch
gekennzeichnet, daß die Aufprallfläche als elektrisches
Heizelement (44) ausgestaltet und mit einem Temperaturfühler (43) versehen ist.

10. Vorrichtung nach Anspruch 9, gekennzeichnet durch
einen zweiten Temperaturfühler (46) zur Anzeige der
Temperatur des angefeuchteten Beatmungsluftgemisches,
welches dem Patienten zugeführt wird.

11. Vorrichtung nach Anspruch 10, gekennzeichnet durch
einen dritten Temperaturfühler (47) zur Regelung der
Temperatur des angefeuchteten Beatmungsluftgemisches,
welches dem Patienten zugeführt wird.

FIG.1

Jet-Ventile

Netzteil

Jet-Steuerung

Druck-Reservoir

Druck-Reservoir

?0V/AC

10

30

26

28

32

34

12

24

22

A D r u c k ⊗
I ⊗
a N e t z
r
m Pause

Kontr. ⊗

1 2 3 4 5 6

Jet-Ventile

Mischer

O₂

20

5 bar

Netz

Start

Stop

14

Frequenz
( 0-300 min⁻¹)

16

Insp.
(10-89 %)

18

19

Betriebsdruck
( 0-5 bar )

( 21-100 % O₂ )

Druckluft

1/3

0058864

36

40

Tubus von vorne

36

40

# FIG. 2

FIG. 3